# EUROPEAN PATENT APPLICATION

(11) **EP 4 697 344 A1**
(43) Date of publication of application: **18.02.2026**
(21) Application number: 24202668.0
(22) Date of filing: 25.09.2024
(51) Int. Cl.: G16H 20/40, A61N 5/10

(54) **METHODS, APPARATUS AND COMPUTER-READABLE MEDIA RELATING TO RADIOTHERAPY TREATMENT PLANNING**

(30) Priority: 16.08.2024 CN 202411131904
(71) Applicant: Elekta, Inc., Atlanta, GA 30346 (US)
(72) Inventor: LIANG, Edward, Atlanta, 30346 (US); CHEN, Charles, Atlanta, 30346 (US); WANG, Qunxi Aaron, Atlanta, 30346 (US)
(74) Representative: Haseltine Lake Kempner LLP

(57) **Abstract**

A treatment-planning apparatus for radiotherapy, the apparatus comprising: processing circuitry; a network interface; and a non-transitory computer-readable medium storing instructions which, when executed by the processing circuitry, cause the processing circuitry to: in a first mode of operation, receive input data comprising imaging data and one or more medical objectives, and perform an optimization process using the input data to generate a treatment plan; and, in a second mode of operation, output data comprising imaging data and one or more medical objectives to an external treatment-planning server via the network interface, and receive a treatment plan from the external treatment-planning server via the network interface.

## Description

### Technical field

The present disclosure relates to a treatment-planning apparatus for radiotherapy treatment planning. The present disclosure also relates to computer-implemented methods for radiotherapy treatment planning, and to a computer program product configured, when run on a computer or processing circuitry, to carry out the methods described herein.

### Background

Radiation therapy or radiotherapy may be described as the use of ionising radiation to damage or destroy unhealthy cells in both humans and animals. The ionising radiation may be directed to tumours on the surface of the skin or deep inside the body. Common forms of ionising radiation include X-rays and charged particles. An example of a radiotherapy technique is Gamma Knife^{®}, where a patient is irradiated using a number of lower-intensity gamma rays that converge with higher intensity and high precision at a targeted region (e.g., a tumour). Another example of radiotherapy comprises using a linear accelerator ("linac"), whereby a targeted region is irradiated by high-energy particles (e.g., electrons, high-energy photons, and the like). In another example, radiotherapy may be provided using a heavy charged particle accelerator (e.g., protons, carbon ions, and the like).

The placement and dose of the radiation beam may be accurately controlled to provide a prescribed dose of radiation to the target region (e.g., the tumour) and to reduce damage to surrounding healthy tissue (known as organs at risk or OARs). The process of determining the placement and dose of the radiation beam(s) for any given treatment is known as treatment planning, and the apparatus which carries out this process is known as a treatment-planning system.

An aspect of treatment planning concerns determining suitable characteristics of radiation to be delivered to produce a safe and effective dose. Characteristics of radiation relate to, for example, a fluence pattern or distribution. The fluence pattern may be dependent on beam arrangements, energies, and field sizes, which are in turn related to controllable parameters (which are optimizable). By determining suitable values for those parameters, a suitable fluence pattern may be obtained.

A radiation therapy treatment plan (treatment plan, or simply plan) may be established using an optimization procedure to determine a set of optimum parameter values or optimum variable values that are expected to deliver a suitable dose. The optimization procedure may be based on clinical and dosimetric objectives and constraints. Examples of clinical and dosimetric objectives and constraints include maximum, minimum, and mean doses to target regions and surrounding regions (e.g., tumours and critical organs). Clinical and dosimetric objectives and constraints may be referred to as treatment planning objectives. Optimization is usually carried out with respect to one or more treatment plan parameters to reduce beam-on time, improve dose uniformity, etc.

A treatment planning procedure may include using an image (two- or three-dimensional) of the patient to identify a target region and to identify critical organs near the target region. The target region (or area to be treated, e.g., a planned target volume, PTV), and surrounding region (e.g., OARs) may be identified using segmentation. After segmentation, a dose plan may be created for the patient indicating the desired amount of radiation to be received by the target region and/or the surrounding region. The target region may have an irregular volume and may be distinctive in terms of its size, shape, and position.

In a practical example, multiple anatomical structures (target regions and/or surrounding regions) may be present. For example, in a head and neck treatment, there may be over 20 anatomical structures. For each structure, compliance with various treatment-planning objectives may be desired. For example, the target region may be associated with a minimum dose objective (in other words, the dose delivered to the target region should be at least X); an OAR may be associated with a maximum dose objective (in other words, the dose delivered to the OAR should be no more than Y). Structures and their objectives may be assigned different priorities in order to achieve a clinically acceptable plan.

Creation of a radiation treatment plan is typically a time-consuming process where a planner may try to comply with various treatment objectives or constraints - considering their individual importance - to produce a radiation treatment plan that is clinically acceptable. Common issues faced when creating radiation treatment plans that involve optimization procedures include lengthy optimization times and high computational burdens required to achieve safe and satisfactory results. These processes often involve trial-and-error on the part of the user (e.g., a treatment planner, dosimetrist, clinician, or health care worker), may be time-consuming, and are further complicated by the addition of further objectives and constraints.

The software used for radiotherapy treatment planning is therefore also complex, and subject to significant ongoing research and development. As new features are added and optimization processes are improved, it can be expected that the software will benefit from updates to new versions. The treatment-planning system, on which such software is installed, is usually located in close proximity to the radiotherapy apparatus on which the treatment plan is to be delivered. For example, it may be located in the same medical centre or medical department. While processes for updating software remotely are well known in a general sense, the patient safety aspects of radiotherapy (and therefore treatment planning) mean that updating treatment planning software is less straightforward. For example, safety checks may need to be carried out once a new version of the software is installed, prior to its use.

An alternative approach is to use a client-server architecture for treatment planning. In this approach, the software installed locally at or near the radiotherapy apparatus comprises only a client which interacts with a remote server to perform optimization tasks. The client is unable to perform optimization tasks itself, but instead communicates with the remote server by transmitting data necessary to generate the treatment plan, and then receiving the treatment plan from the remote server. The remote server may provide treatment-planning services to multiple clients, and the treatment-planning software installed at the remote server can be updated as new versions are made available. Some problems with this approach are that it relies on adequate performance by the remote server, and a reliable network between the client and the server over which to communicate. If the network between the client and the server fails, treatment planning cannot continue and ultimately the radiotherapy apparatus is unable to be used.

An improved system for radiotherapy treatment planning is therefore desirable.

### Summary

It is an object of embodiments of the disclosure to address these and other problems in the art.

The invention is defined in the independent claims, to which reference should now be made. Further features are set out in the dependent claims.

In one aspect, the disclosure provides a treatment-planning apparatus for radiotherapy. The apparatus comprises: processing circuitry; a network interface; and a non-transitory computer-readable medium. The medium stores instructions which, when executed by the processing circuitry, cause the processing circuitry to: in a first mode of operation, receive input data comprising imaging data and one or more medical objectives, and perform an optimization process using the input data to generate a treatment plan; and, in a second mode of operation, output data comprising imaging data and one or more medical objectives to an external treatment-planning server via the network interface, and receive a treatment plan from the external treatment-planning server via the network interface.

In a second aspect, the disclosure provides a computer-implemented method performed by a computer or processing circuitry for radiotherapy treatment planning. The method comprises: in a first mode of operation, receiving input data comprising imaging data and one or more medical objectives, and performing an optimization process using the input data to generate a treatment plan; and, in a second mode of operation, outputting data comprising imaging data and one or more medical objectives to an external treatment-planning server via a network interface, and receiving a treatment plan from the external treatment-planning server via the network interface.

In a third aspect, the disclosure provides a computer program product for performing the method of the second aspect. The computer program product may comprise a computer-readable medium, the computer readable medium having computer-readable code embodied therein, the computer-readable code being configured such that, on execution by a computer or processing circuitry, the computer or processing circuitry is caused to: in a first mode of operation, receive input data comprising imaging data and one or more medical objectives, and perform an optimization process using the input data to generate a treatment plan; and, in a second mode of operation, output data comprising imaging data and one or more medical objectives to an external treatment-planning server via a network interface, and receive a treatment plan from the external treatment-planning server via the network interface..

### Brief Description of the Drawings

For a better understanding of the present disclosure, and to show more clearly how it may be carried into effect, reference will now be made, by way of example, to the following drawings in which:
Figure 1 is a schematic diagram of a radiotherapy treatment-planning system according to embodiments of the disclosure;
Figure 2 is a flowchart of a computer-implemented method according to embodiments of the disclosure;
Figure 3 is a schematic diagram of a radiotherapy treatment-planning apparatus according to embodiments of the disclosure; and
Figure 4 is a schematic diagram of a radiotherapy delivery apparatus.

### Detailed Description

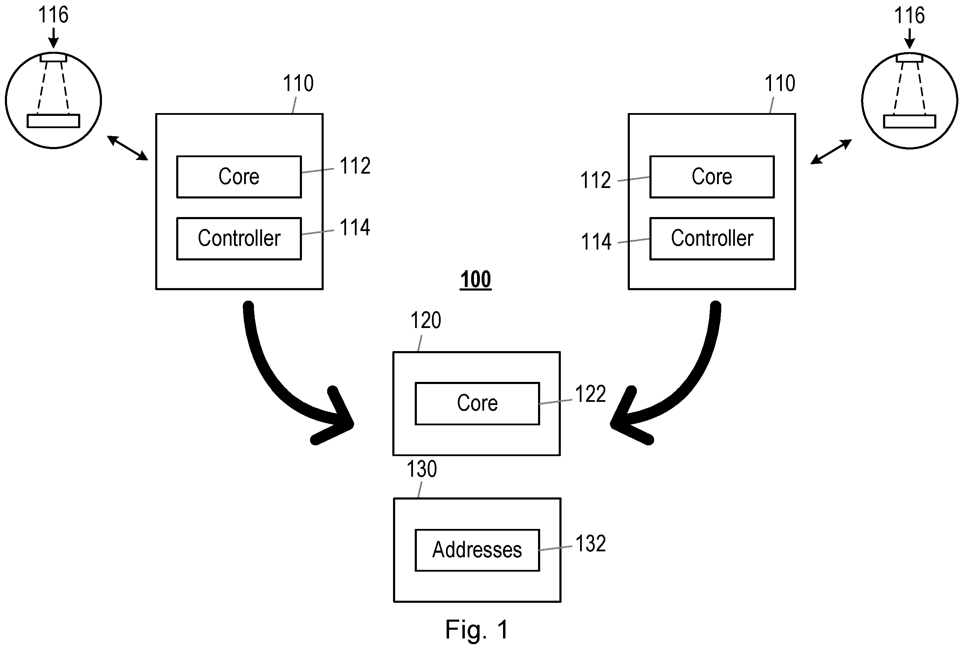

Figure 1 is a schematic diagram of a radiotherapy treatment-planning system 100 according to embodiments of the disclosure. The system 100 comprises one or more local treatment-planning apparatuses 110 (in the illustrated embodiment the system 100 comprises a plurality of such apparatuses 110), and a central treatment-planning server 120. In some embodiments, the system 100 further comprises an addressing server 130.

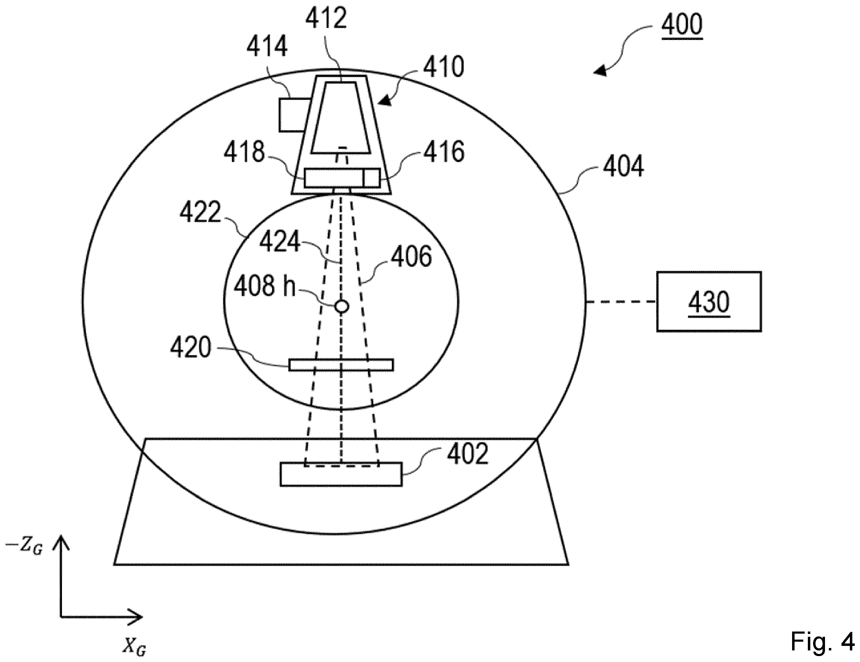

Each treatment-planning apparatus 110 is associated with one or more radiotherapy delivery apparatuses 116, and provides treatment plans to those radiotherapy delivery apparatuses. One possible radiotherapy delivery apparatus is shown schematically in Figure 4, although those skilled in the art will appreciate that different radiotherapy methodologies and architectures are possible within the scope of the present disclosure.

Each treatment-planning apparatus 110 may be described as "local" in the sense that it is in close proximity to the one or more radiotherapy delivery apparatuses 116 on which the treatment plans provided by the treatment-planning apparatus 110 are to be delivered. For example, the treatment-planning apparatus 110 may be located in the same medical centre or medical department as the one or more radiotherapy delivery apparatuses. In another example, the treatment-planning apparatus 110 may be located on a same local communication network (e.g., LAN) as the one or more radiotherapy delivery apparatuses 116. In some embodiments, the treatment-planning apparatus 110 may be co-located with the radiotherapy delivery apparatus 116. For example, the treatment-planning apparatus 110 may be implemented within the same computing system or controller that controls the radiotherapy delivery apparatus (see below with respect to Figure 4).

The treatment-planning apparatus 110 comprises a treatment planning core 112 (also referred to as a "core") and a treatment-planning controller 114 (also referred to as a "controller"). The core 112 comprises hardware, software or a combination of hardware and software, that is configured to perform treatment planning processes. For example, the core may comprise a dedicated processor for performing treatment planning processes, or a general-purpose processor which runs software for performing treatment planning processes.

Those skilled in the art will appreciate that the precise nature of the treatment planning processes performed by the core 112 are not relevant for an understanding of embodiments of the disclosure. In general, however, treatment planning processes may comprise one or more optimization processes to determine a treatment plan. For example, such processes may determine the weights of one or more radiation beamlets delivered as part of the radiotherapy. The optimization processes may utilize a cost function comprising a mathematical expression relating a dose distribution (e.g., at unit fluence) to the beamlet weights. The cost function may incorporate one or more medical objectives (also referred to as reference objectives) and/or one or more constraints. The reference objectives may set out one or more of a maximum dose, a minimum dose and a mean dose to be achieved within a given volume (e.g., target, OAR, healthy tissue, etc). The cost function may be configured so as to take a higher value to reflect that a given dose distribution fails to meet one or more of those objectives, and a lower value to reflect that one or more of those objectives are met by a given dose distribution. The constraints may be implemented by setting hard constraints on the output parameters (e.g., the beamlet weights), to reflect the physical reality and/or constraints of the radiotherapy apparatus 116 that is to deliver the therapy. For example, a basic constraint may be that the beamlet weights cannot be negative. Further constraints may impose a maximum beamlet weight, e.g., as defined by a maximum power output of the radiotherapy apparatus 116, or permitted angular ranges from which the radiation can be delivered, e.g., as defined by the physical geometry of the radiotherapy apparatus 116.

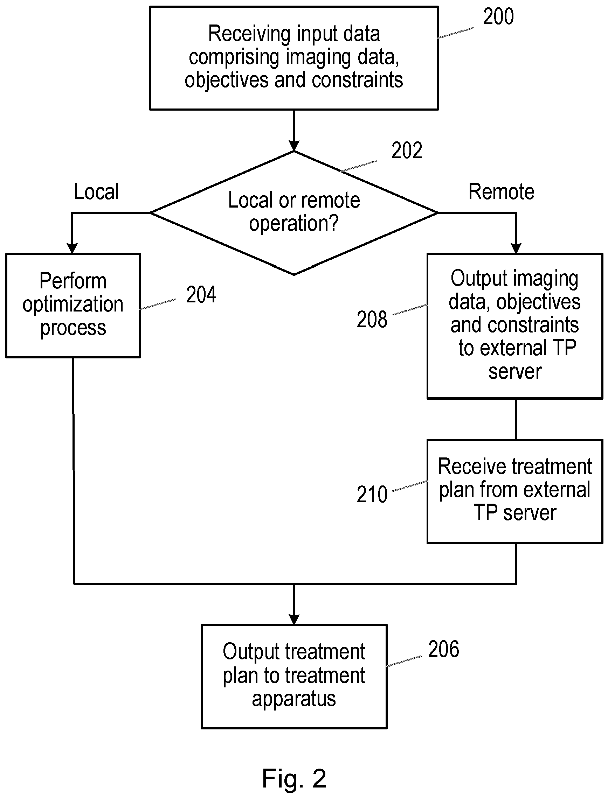

The controller 114 is responsible for determining the mode of operation of the treatment-planning apparatus 110, and for controlling a treatment planning process in accordance with that mode of operation. In a first mode, treatment planning is performed within the treatment-planning apparatus; that is, the treatment-planning apparatus 110 generates a treatment plan using its own core 112. In a second mode, the treatment-planning apparatus communicates with the central treatment-planning server 120 and receives the treatment plan from that central server 120. Importantly, the apparatus 110 has the capability to operate in both modes of operation. That is, it comprises the core 112, which enables the apparatus to generate treatment plans itself (i.e., in the first mode of operation), and it possesses one or more interfaces to communicate with the central server 120 and to obtain a treatment plan by that approach (i.e., in the second mode of operation). Further detail regarding this operation is set out below with respect to the method shown in Figure 2.

The central treatment-planning server 120 may be described as "central" to distinguish it from the "local" treatment-planning apparatuses 110. Those skilled in the art will appreciate that there is no requirement for the treatment-planning server 120 to be centrally placed within the system 100 in a geographical sense or any other sense. The central treatment-planning server 120 is external to, and remote from, a local treatment-planning apparatus 110 that communicates with it to generate a treatment plan in accordance with the second mode of operation, discussed in greater detail with respect to Figure 2 below.

The central treatment-planning server 120 comprises its own treatment-planning core 122. This core 122 may be substantially similar to the core 112 implemented in the local treatment planning apparatus 110. That is, the core 122 also comprises hardware, software or a combination of hardware and software, that is configured to perform treatment planning processes. The core may comprise a dedicated processor for performing treatment planning processes, or a general-purpose processor which runs software for performing treatment planning processes. The central treatment-planning server 120 may be operable to perform treatment-planning processes for multiple local treatment-planning apparatuses 110, and therefore the core 122 may have a greater capacity than the core 112. For example, the core 122 may be implemented in multiple processors, multiple servers, processors with greater computing power, etc.

In the illustrated embodiment, the central treatment-planning server 120 is not associated with any particular radiotherapy apparatus. That is, it is the role of the central treatment-planning server 120 to provide treatment plans to the local treatment-planning apparatuses 110, for implementation by their associated radiotherapy apparatuses 116; the central treatment-planning server 120 does not generate treatment plans for implementation by any radiotherapy apparatus that is associated with itself. However, in other embodiments, the central treatment-planning server 120 may have one or more associated radiotherapy apparatuses in a similar manner to the local treatment-planning apparatuses 110. In such embodiments, the central treatment-planning server 120 may be substantially similar to the local treatment-planning apparatus 110, except for providing an external treatment-planning service for other treatment-planning apparatuses 110.

The addressing server 130 comprises a database 132 of addressing information (e.g., Internet Protocol addresses) for the central treatment-planning server 120, and potentially also one or more or all of the local treatment planning apparatuses 110.

As noted above, while processes for updating software remotely are well known in a general sense, the patient safety aspects of radiotherapy (and therefore treatment planning) mean that updating treatment planning software is less straightforward. For example, safety checks may need to be carried out once a new version of the software is installed, prior to its use. However, this approach relies on adequate performance by the remote server, and a reliable network between the client and the server over which to communicate. If the network between the client and the server fails, treatment planning cannot continue and ultimately the radiotherapy apparatus is unable to be used.

Embodiments of the disclosure address this problem by providing a treatment-planning apparatus that is selectively operable in one of two operating modes. In a first mode, treatment planning is performed within the treatment-planning apparatus; that is, in the context of the system 100 described above, the treatment-planning apparatus 110 generates a treatment plan using its own core 112. In a second mode, the treatment-planning apparatus communicates with a remote treatment-planning server (e.g., in a client-server architecture) and receives the treatment plan from the remote treatment-planning server; in the context of the system 100, the treatment-planning apparatus 110 transmits input data to the central treatment-planning server 120, which generates the treatment plan and provides it back to the treatment-planning apparatus 110 for implementation by the radiotherapy system 116. Further detail is set out below with respect to Figure 2.

**Figure 2** is a flowchart of a computer-implemented method according to embodiments of the disclosure. The method may be implemented by a treatment planning apparatus, such as the local treatment-planning apparatus 110 shown in Figure 1.

The method beings in step 200, in which the apparatus receives input data with which to generate a treatment plan. For example, the input data may comprise imaging data (e.g., two-dimensional or three-dimensional imaging data) of a patient for whom the treatment plan is to be generated. The input data may further comprise one or more medical objectives to be met by the treatment plan, e.g., one or more of a maximum dose, a minimum dose and a mean dose to be achieved within a given volume (e.g., target, OAR, healthy tissue, etc). The input data may further comprise an indication of one or more constraints for the treatment-planning process, e.g., based on a radiotherapy apparatus that is to implement the treatment plan (e.g., a maximum dose rate, ranges of possible angles of delivery of a radiation beam, etc), and/or based on physical reality (e.g., beamlet weights cannot be negative).

The input data may be received directly from an imaging system (e.g., magnetic-resonance imaging data, computational tomography data, etc), or from an intermediate database storing such data. The medical objectives and/or constraints may similarly be received direct from a medical operator of the treatment-planning system, or from a database storing such data (and provided initially by a physician or other medically qualified person).

In step 202, the apparatus determines whether it is operating in a first mode of operation (also referred to as a "local" mode of operation herein) or a second mode of operation (also referred to as a "remote" mode of operation).

Which of the first and second modes of operation is selected may be the subject of user input and/or automated input. For example, a user of the treatment-planning apparatus may select or configure the apparatus to operate in the first or second mode of operation. One or other of the modes of operation may be selected as a default mode, that is to be utilized in the absence of any other user input; for example, the first mode of operation may be a default mode.

The mode of operation may be selected based on one or more performance criteria. In particular, as the second mode of operation relies on adequate performance of the external treatment planning server, the first mode of operation may be selected (e.g., automatically) responsive to a determination that the external treatment-planning server does not meet one or more performance criteria. Such performance criteria may comprise one or more of: the external treatment-planning server being reachable via the network interface; the external treatment-planning server generating the treatment plan within a threshold time; the external treatment-planning server having a workload less than a threshold amount. That is, if the external treatment-planning server becomes unreachable (e.g., through a failure of the network), then the first mode of operation may be selected; failures in the network may be detected through time-out of attempts to reach the external treatment-planning server by the apparatus. If the external treatment-planning server has a high workload, or fails to generate treatment plans within an adequate timeframe, the first mode of operation may also be selected. Performance statistics (and/or current workload levels) may be shared by the external treatment-planning server with the apparatus, or measured by the apparatus itself, and then used as part of the selection process.

The first mode of operation is a standalone mode, in which the local treatment planning apparatus (e.g., the core 112) generates a treatment plan based on the input data without outputting imaging data or medical objectives and/or constraints over a network interface to the central server 120. Optimization and/or calculation tasks are performed locally by the core 112. The first mode of operation may correspond to local inter-process communication (IPC).

In the second mode of operation, optimization and/or calculation tasks are performed by the external treatment-planning server. The second mode of operation may correspond to remote procedural call (RPC), with the local treatment planning apparatus 110 operating as a client and the external treatment planning server 120 operating as a server.

In the first mode of operation, the process moves to step 204, with the local treatment planning apparatus itself performing optimization and/or calculation tasks, using the input data received in step 200, to generate a treatment plan. Thus the controller 114 directs the core 112 to carry out the optimization and/or calculation tasks, using the input data received in step 200, to generate a treatment plan.

In step 206, the treatment plan so generated is output to the radiotherapy delivery apparatus 116 to be implemented. Alternatively, the treatment plan may be output to a database for storage, to be implemented by the apparatus 116 at a later time.

In the second mode of operation, the process moves to step 208, in which the local treatment planning apparatus outputs imaging data, medical objectives and/or constraints over its network interface to the external treatment planning server 120. The local treatment planning apparatus may also output an instruction to the external treatment planning server 120 to perform one or more optimization and/or calculation tasks using the output data, in order to generate a treatment plan. That is, the local treatment planning apparatus causes a procedure (e.g., subroutine) to be executed on the external treatment planning server.

The local treatment-planning apparatus may communicate with an addressing server (e.g., addressing server 130) in order to discover the address of the external server 120. Where the system 100 comprises multiple external treatment planning servers, the addressing server 130 may implement one or more load-balancing algorithms to identify a server 120 with the best capacity to handle the request from the local treatment-planning apparatus. Once identified, the addressing server 130 provides address information for the identified server 120 to the apparatus 110, so that communication can take place between the two entities.

The external treatment-planning server 120 thus performs optimization and/or calculation tasks (e.g., using its core 122) to generate a treatment plan, and in step 210 the apparatus 110 receives the treatment plan from the external server 120. The process moves to step 206, and the treatment plan so received is output to the radiotherapy delivery apparatus 116 to be implemented. Alternatively, the treatment plan may be output to a database for storage, to be implemented by the apparatus 116 at a later time.

Communication between the apparatus 110 and the server 120 may be direct or indirect. In the former case, the data transmitted in step 208 and/or the treatment plan received in step 210 may be passed directly between the apparatus 110 and the server 120 (e.g., without intermediate network nodes between the two entities). In the latter case, the data transmitted in step 208 and/or the treatment plan received in step 210 may be passed between the two entities via one or more intermediate network nodes. The intermediate network nodes for the data transmitted in step 208 and the for the treatment plan received in step 210 may be the same or different.

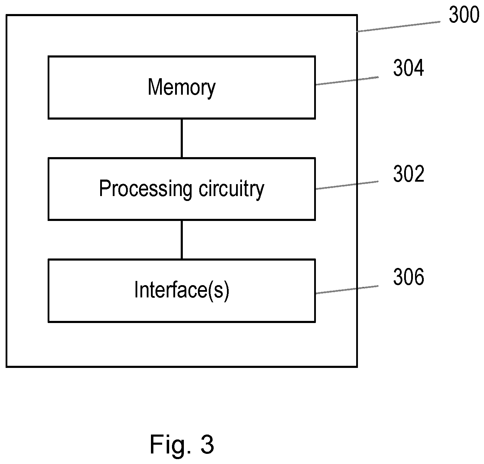

**Figure 3** is a schematic diagram of a radiotherapy treatment-planning apparatus 300 according to embodiments of the disclosure. The optical network element comprises processing circuitry 302, a non-transitory computer readable medium 304 and one or more interfaces 306.

The processing circuitry 302 may comprise a combination of one or more of a microprocessor, controller, microcontroller, central processing unit, digital signal processor, application-specific integrated circuit, field programmable gate array, or any other suitable computing device, resource, or combination of hardware, software and/or encoded logic operable to provide, either alone or in conjunction with other components of the treatment planning system 300, such as the memory 304, functionality of the treatment planning system 300. For example, the processing circuitry 302 may be configured to cause the treatment planning system to perform the method as described with reference to Figure 2.

Thus, in one embodiment, the processing circuitry 302 is configured to cause the treatment planning system 300 to: in a first mode of operation, receive input data comprising imaging data and medical objectives, and perform an optimization process using the input data to generate a treatment plan; and, in a second mode of operation, output data comprising imaging data and medical objectives to an external treatment-planning server via the network interface, and receive a treatment plan from the external treatment-planning server via the network interface.

The memory 304 may comprise any form of volatile or non-volatile computer-readable memory including, without limitation, persistent storage, solid-state memory, remotely mounted memory, magnetic media, optical media, random access memory (RAM), read-only memory (ROM), mass storage media (for example, a hard disk), removable storage media (for example, a flash drive, a Compact Disk (CD) or a Digital Video Disk (DVD)), and/or any other volatile or non-volatile, non-transitory device-readable and/or computer-executable memory devices that store information, data, and/or instructions that may be used by the processing circuitry 302. The memory 304 may store any suitable instructions, data, or information, including a computer program, software, an application including one or more of logic, rules, code, tables, and/or other instructions capable of being executed by the processing circuitry 302 and utilized by the treatment planning system 300 (e.g., for performing the method of Figure 2). The memory 304 may be used to store any calculations made by the processing circuitry 302 and/or any data received via the communication interface 306. In some embodiments, the processing circuitry 302 and memory 304 are integrated.

The communication interface(s) 306 are used in wired or wireless communication of signaling and/or data between the treatment planning system 300 and a network, and/or between the treatment planning system 300 and one or more network servers (such as the central planning server 120 and/or the addressing server 130). The communication interface 306 may comprises port(s)/terminal(s) to send and receive data, for example to and from a network over a wired connection (e.g., an electrical, optical, etc) and/or a wireless connection.

The methods of the present disclosure may be implemented in hardware, or as software modules running on one or more processors. The methods may also be carried out according to the instructions of a computer program, and the present disclosure also provides a computer readable medium having stored thereon a program for carrying out any of the methods described herein. A computer program embodying the disclosure may be stored on a computer readable medium, or it could, for example, be in the form of a signal such as a downloadable data signal provided from an Internet website, or it could be in any other form.

**Figure 4** depicts a radiotherapy delivery apparatus 400, suitable for implementing radiation treatment plans determined according to embodiments of the disclosure.

The radiotherapy apparatus 400 comprises a radiation head 410 and a beam receiving apparatus 402, both of which are attached to a gantry 404. The radiation head 410 includes a radiation source 412, which emits a beam of radiation 406. The radiation source may comprise a linear accelerator, designed to accelerate charged particles (e.g., protons, electrons, etc) to therapeutic energies (e.g., in the range of MeV). The radiation so generated may include the charged particles themselves (e.g., alpha or beta radiation) and/or secondary radiation (e.g., x-rays, neutrons, etc) generated by directing the charged particles towards a target. The radiation head 410 also includes a beam shaping apparatus 418, which controls the size and shape of the radiation field associated with the beam.

The beam receiving apparatus 402 is configured to receive radiation emitted from the radiation head 410, for the purpose of absorbing and/or measuring the beam of radiation. In the view shown, the radiation head 410 and the beam receiving apparatus 402 are positioned diametrically opposed to one another.

The gantry 404 may be rotatable, and support the radiation head 410 and the beam receiving apparatus 402 such that they are rotatable around an axis of rotation 408, which may coincide with the patient longitudinal axis. The gantry provides rotation of the radiation head 410 and the beam receiving apparatus 402 in a plane perpendicular to the patient longitudinal axis (e.g., a sagittal plane). Three gantry directions X_{G}, Y_{G}, Z_{G} may be defined, where the Y_{G} direction is perpendicular with gantry axis of rotation. The Z_{G} direction extends from a point on the gantry corresponding to the radiation head, towards the axis of rotation of the gantry. Therefore, from the patient frame of reference, the Z_{G} direction rotates around as the gantry rotates.

Radiotherapy apparatus 400 also includes a support surface 420 on which a subject (or patient) is supported during radiotherapy treatment. The radiation head 410 is configured to rotate around the axis of rotation 408 such that the radiation head 410 directs radiation towards the subject from various angles around the subject in order to spread out the radiation dose received by healthy tissue to a larger region of healthy tissue while building up a prescribed dose of radiation at a target region.

The radiotherapy apparatus 400 is configured to deliver a radiation beam towards a radiation isocentre, which is substantially located on the axis of rotation 408 at the centre of the gantry 404 regardless of the angle at which the radiation head 410 is placed.

The rotatable gantry 404 and radiation head 410 are dimensioned so as to allow a central bore 422 to exist. The central bore 422 provides an opening, sufficient to allow a subject to be positioned therethrough without the possibility of being incidentally contacted by the radiation head 410 or other mechanical components as the gantry rotates the radiation head 410 about the subject.

The radiation head 410 emits the radiation beam 406 along a beam axis 424 (or radiation axis or beam path), where the beam axis 424 is used to define the direction in which the radiation is emitted by the radiation head. The radiation beam 406 is incident on the beam receiving apparatus 402, which may include at least one of a beam stopper and a radiation detector. The beam receiving apparatus 402 is attached to the gantry 404 on a diametrically opposite side to the radiation head 410 to attenuate and/or detect a beam of radiation after the beam has passed through the subject.

The radiation beam axis 424 may be defined as, for example, a centre of the radiation beam 406 or a point of maximum intensity.

The beam shaping apparatus 418 delimits the spread of the radiation beam 406. The beam shaping apparatus 418 is configured to adjust the shape and/or size of a field of radiation produced by the radiation source. The beam shaping apparatus 418 does this by defining an aperture (also referred to as a window or an opening) of variable shape to collimate the radiation beam 406 to a chosen cross-sectional shape. In this example, the beam shaping apparatus 418 may be provided by a combination of a diaphragm and an MLC. Beam shaping apparatus 418 may also be referred to as a beam modifier.

The radiotherapy apparatus 400 may be configured to deliver both coplanar and non-coplanar (also referred to as tilted) modes of radiotherapy treatment. In coplanar treatment, radiation is emitted in a plane which is perpendicular to the axis of rotation of the radiation head 410. In non-coplanar treatment, radiation is emitted at an angle which is not perpendicular to the axis of rotation. In order to deliver coplanar and non-coplanar treatment, the radiation head 410 may move between at least two positions, one in which the radiation is emitted in a plane which is perpendicular to the axis of rotation (coplanar configuration) and one in which radiation is emitted in a plane which is not perpendicular to the axis of rotation (non-coplanar configuration).

In the coplanar configuration, the radiation head is positioned to rotate about a rotation axis and in a first plane. In the non-coplanar configuration, the radiation head is tilted with respect to the first plane such that a field of radiation produced by the radiation head is directed at an oblique angle relative to the first plane and the rotation axis. In the non-coplanar configuration, the radiation head is positioned to rotate in a respective second plane parallel to and displaced from the first plane. The radiation beam is emitted at an oblique angle with respect to the second plane, and therefore as the radiation head rotates the beam sweeps out a cone shape.

The beam receiving apparatus 402 remains in the same place relative to the rotatable gantry when the radiotherapy apparatus is in both the coplanar and non-coplanar modes. Therefore, the beam receiving apparatus 402 is configured to rotate about the rotation axis in the same plane in both coplanar and non-coplanar modes. This may be the same plane as the plane in which the radiation head rotates.

The beam shaping apparatus 410 is configured to reduce the spread of the field of radiation in the non-coplanar configuration in comparison to the coplanar configuration.

The radiotherapy apparatus 400 includes a controller 430, which is programmed to control the radiation source 412, beam receiving apparatus 806 and the gantry 802. Controller 430 may perform functions or operations such as treatment planning, treatment execution, image acquisition, image processing, motion tracking, motion management, and/or other tasks involved in a radiotherapy process. That is, in one embodiment, the local treatment-planning apparatus 110 may be implemented within the controller 430 of the radiotherapy delivery apparatus 400. In other embodiments, the local treatment-planning apparatus 110 may be implemented in a separate computing device.

Controller 430 is programmed to control features of apparatus 400 according to a radiotherapy treatment plan for irradiating a target region, also referred to as a target tissue, of a patient. The treatment plan includes information about a particular dose to be applied to a target tissue, as well as other parameters such as beam angles, dose-histogram-volume information, the number of radiation beams to be used during therapy, the dose per beam, and the like. Controller 430 is programmed to control various components of apparatus 400, such as gantry 404, radiation head 410, beam receiving apparatus 402, and support surface 420, according to the treatment plan.

Hardware components of controller 430 may include one or more computers (e.g., general purpose computers, workstations, servers, terminals, portable/mobile devices, etc.); processors (e.g., central processing units (CPUs), graphics processing units (GPUs), microprocessors, digital signal processors (DSPs), field programmable gate arrays (FPGAs), special-purpose or specially-designed processors, etc.); memory/storage devices such as a memory (e.g., read-only memories (ROMs), random access memories (RAMs), flash memories, hard drives, optical disks, solid-state drives (SSDs), etc.); input devices (e.g., keyboards, mice, touch screens, mics, buttons, knobs, trackballs, levers, handles, joysticks, etc.); output devices (e.g., displays, printers, speakers, vibration devices, etc.); circuitries; printed circuit boards (PCBs); or other suitable hardware. Software components of controller 430 may include operation device software, application software, etc.

The radiation head 410 may be connected to a head actuator 414, which is configured to actuate the radiation head 410, for example between a coplanar configuration and one or more non-coplanar configurations. This may involve translation and rotation of the radiation head 410 relative to the gantry. In some implementations, the head actuator may include a curved rail along which the radiation head 410 may be moved to adjust the position and angle of the radiation head 410. The controller 430 may control the configuration of the radiation head 430 via the head actuator 414.

The beam shaping apparatus 418 includes a shaping actuator 416. The shaping actuator is configured to control the position of one or more elements in the beam shaping apparatus 418 in order to shape the radiation beam 406. In some implementations, the beam shaping apparatus 418 includes an MLC, and the shaping actuator 416 includes means for actuating leaves of the MLC. The beam shaping apparatus 418 may further comprise a diaphragm, and the shaping actuator 416 may include means for actuating blocks of the diaphragm. The controller 430 may control the beam shaping apparatus 418 via the shaping actuator 416.

A treatment plan may comprise positioning information of beam shaping apparatus 418. The positioning information of beam shaping apparatus 418 may comprise information indicating a configuration of one or more elements of beam shaping apparatus 418, such as leaf configuration of an MLC of beam shaping apparatus 418, a configuration of a diaphragm of beam shaping apparatus 418, a configuration of an opening (e.g., window or aperture) of the MLC, and/or the like.

Those skilled in the art will appreciate that the apparatus 400 is just one example of a radiotherapy apparatus on which treatment plans may be delivered. Alternative radiotherapy delivery apparatuses may not include one or more of the features described with respect to Figure 4, or may be configured differently (e.g., the source 412 may be fixed in position, or the apparatus may comprise a plurality of radiation sources configured to deliver radiation from a plurality of directions). In general, embodiments of the present disclosure are applicable to any radiotherapy delivery apparatus which requires the generation of a treatment plan.

Unless specifically stated otherwise, as apparent from the discussion above, it is appreciated that throughout the description, discussions utilizing terms such as "receiving", "determining", "comparing", "enabling", "maintaining," "identifying", "obtaining", "accessing" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

While certain embodiments have been described, these embodiments have been presented by way of example only, and are not intended to limit the scope of the present disclosure. Indeed, the novel methods and apparatuses described herein may be embodied in a variety of other forms; furthermore, various omissions, substitutions and changes in the form of methods and apparatus described herein may be made.

For the avoidance of doubt, the following numbered paragraphs set out embodiments of the disclosure:
1. A treatment-planning apparatus for radiotherapy, the apparatus comprising:
   processing circuitry;
   a network interface; and
   a non-transitory computer-readable medium storing instructions which, when executed by the processing circuitry, cause the processing circuitry to:
      in a first mode of operation, receive input data comprising imaging data and one or more medical objectives, and perform an optimization process using the input data to generate a treatment plan, and
      in a second mode of operation, output data comprising imaging data and one or more medical objectives to an external treatment-planning server via the network interface, and receive a treatment plan from the external treatment-planning server via the network interface.
2. The treatment-planning apparatus of embodiment 1, wherein the processing circuitry is configurable in the first mode of operation or the second mode of operation based on user input.
3. The treatment-planning apparatus of embodiment 1, wherein the first mode of operation is a default mode.
4.The treatment-planning apparatus of embodiment 1, wherein the processing circuitry is configured in the first mode of operation responsive to a determination that the external treatment-planning server does not meet one or more performance criteria.
5.The treatment-planning apparatus of embodiment 4, wherein the one or more performance criteria comprise one or more of: the external treatment-planning server being reachable via the network interface; the external treatment-planning server generating the treatment plan within a threshold time; the external treatment-planning server having a workload less than a threshold amount.
6.The treatment-planning apparatus of any one of the preceding embodiments, wherein the first mode of operation is a standalone mode, in which the processing circuitry generates the treatment plan without outputting imaging data or medical objectives over the network interface.
7.The treatment-planning apparatus of any one of the preceding embodiments, wherein optimization and/or calculation tasks are performed locally by the processing circuitry in the first mode of operation.
8.The treatment-planning apparatus of any one of the preceding embodiments, wherein the first mode of operation corresponds to local inter-process communication.
9.The treatment-planning apparatus of any one of the preceding embodiments, wherein, in the second mode of operation, optimization and/or calculation tasks are performed by the external treatment-planning server.
10. The treatment-planning apparatus of any one of the preceding embodiments, wherein the second mode of operation corresponds to remote procedural call.
11. The treatment-planning apparatus of any one of the preceding embodiments, wherein, in the second mode of operation, the processing circuitry is operable to obtain address information for the external treatment-planning server via communications with an external addressing server.
12. The treatment-planning apparatus of embodiment 11, wherein, the address information comprises an Internet Protocol address.
13. The treatment-planning apparatus of any one of the preceding embodiments, wherein, in the second mode of operation, the data comprising imaging data and one or more medical objectives is sent to the external treatment-planning server over the network interface and via one or more first intermediate network nodes.
14. The treatment-planning apparatus of any one of the preceding embodiments, wherein, in the second mode of operation, the treatment plan is received from the external treatment-planning server over the network interface via one or more second intermediate network nodes.
15. A computer-implemented method performed by a computer or processing circuitry for radiotherapy treatment planning, the method comprising:
   in a first mode of operation, receiving input data comprising imaging data and one or more medical objectives, and performing an optimization process using the input data to generate a treatment plan, and
   in a second mode of operation, outputting data comprising imaging data and one or more medical objectives to an external treatment-planning server via a network interface, and receiving a treatment plan from the external treatment-planning server via the network interface.
16. The method of embodiment 15, wherein the first mode of operation or the second mode of operation is selected based on user input.
17. The method of embodiment 15, wherein the first mode of operation is a default mode.
18. The method of embodiment 15, wherein the first mode of operation is selected responsive to a determination that the external treatment-planning server does not meet one or more performance criteria.
19. The method of embodiment 18, wherein the one or more performance criteria comprise one or more of: the external treatment-planning server being reachable via the network interface; the external treatment-planning server generating the treatment plan within a threshold time; the external treatment-planning server having a workload less than a threshold amount.
20. The method of any one of embodiments 15 to 19, wherein the first mode of operation is a standalone mode, in which the computer or processing circuitry generates the treatment plan without outputting imaging data or medical objectives over the network interface.
21. The method of any one of embodiments 15 to 20, wherein optimization and/or calculation tasks are performed locally by the computer or processing circuitry in the first mode of operation.
22. The method of any one of embodiments 15 to 21, wherein the first mode of operation corresponds to local inter-process communication.
23. The method of any one of embodiments 15 to 22, wherein, in the second mode of operation, optimization and/or calculation tasks are performed by the external treatment-planning server.
24. The method of any one of embodiments 15 to 23, wherein the second mode of operation corresponds to remote procedural call.
25. The method of any one of embodiments 15 to 24, further comprising, in the second mode of operation, obtaining address information for the external treatment-planning server via communications with an external addressing server.
26. The method of embodiment 25, wherein the address information comprises an Internet Protocol address.
27. The method of any one of embodiments 15 to 26, wherein, in the second mode of operation, the data comprising imaging data and one or more medical objectives is sent to the external treatment-planning server over the network interface and via one or more first intermediate network nodes.
28. The method of any one of embodiments 15 to 27, wherein, in the second mode of operation, the treatment plan is received from the external treatment-planning server over the network interface via one or more second intermediate network nodes.
29. A computer program product comprising a computer-readable medium, the computer readable medium having computer-readable code embodied therein, the computer-readable code being configured such that, on execution by a computer or processing circuitry, the computer or processing circuitry is caused to perform the method of any one of embodiments 15 to 28.

## Claims

1. A computer-implemented method performed by a computer or processing circuitry for radiotherapy treatment planning, the method comprising:
in a first mode of operation, receiving input data comprising imaging data and one or more medical objectives, and performing an optimization process using the input data to generate a treatment plan, and
in a second mode of operation, outputting data comprising imaging data and one or more medical objectives to an external treatment-planning server via a network interface, and receiving a treatment plan from the external treatment-planning server via the network interface.

2. The method of claim 1, wherein the first mode of operation or the second mode of operation is selected based on user input.

3. The method of claim 1, wherein the first mode of operation is a default mode.

4. The method of claim 1, wherein the first mode of operation is selected responsive to a determination that the external treatment-planning server does not meet one or more performance criteria.

5. The method of claim 4, wherein the one or more performance criteria comprise one or more of: the external treatment-planning server being reachable via the network interface; the external treatment-planning server generating the treatment plan within a threshold time; the external treatment-planning server having a workload less than a threshold amount.

6. The method of any one of the preceding claims, wherein the first mode of operation is a standalone mode, in which the computer or processing circuitry generates the treatment plan without outputting imaging data or medical objectives over the network interface.

7. The method of any one of the preceding claims, wherein optimization and/or calculation tasks are performed locally by the computer or processing circuitry in the first mode of operation.

8. The method of any one of the preceding claims, wherein the first mode of operation corresponds to local inter-process communication.

9. The method of any one of the preceding claims, wherein, in the second mode of operation, optimization and/or calculation tasks are performed by the external treatment-planning server.

10. The method of any one of the preceding claims, wherein the second mode of operation corresponds to remote procedural call.

11. The method of any one of the preceding claims, further comprising, in the second mode of operation, obtaining address information for the external treatment-planning server via communications with an external addressing server.

12. The method of any one of the preceding claims, wherein, in the second mode of operation, the data comprising imaging data and one or more medical objectives is sent to the external treatment-planning server over the network interface and via one or more first intermediate network nodes.

13. The method of any one of the preceding claims, wherein, in the second mode of operation, the treatment plan is received from the external treatment-planning server over the network interface via one or more second intermediate network nodes.

14. A treatment-planning apparatus for radiotherapy, the apparatus comprising:
processing circuitry;
a network interface; and
a non-transitory computer-readable medium storing instructions which, when executed by the processing circuitry, cause the processing circuitry to perform the method of any one of claims 1 to 13.

15. A computer program product comprising a computer-readable medium, the computer readable medium having computer-readable code embodied therein, the computer-readable code being configured such that, on execution by a computer or processing circuitry, the computer or processing circuitry is caused to perform the method of any one of claims 1 to 13.
